# EUROPEAN PATENT APPLICATION

(11) **EP 4 755 404 A1**
(43) Date of publication of application: **10.06.2026**
(21) Application number: 25220345.0
(22) Date of filing: 03.12.2025
(51) Int. Cl.: A61L 2/07, A61L 2/22, A61L 11/00, B05B 1/00, B08B 9/08

(54) **SANITIZATION APPARATUS FOR A CONTAINER DESIGNED FOR THE TRANSPORT OF POTENTIALLY INFECTED OR CONTAMINATED MATERIAL**

(30) Priority: 03.12.2024 IT 202400027354
(71) Applicant: Ecomade Engineering Srl, 35042 Este (PD) (IT)
(72) Inventor: Formaggio, Daniele, Este (PD) (IT)
(74) Representative: De Giorgi, Michele

(57) **Abstract**

Method for sanitizing a container (11) for transporting potentially infected or contaminated material which comprises:
- step A, of housing the container (11) in the sanitization area (C) of a sanitization apparatus (10) according to one of the preceding claims.
- a step B, following step A, of spraying the container (11) with the sanitizing fluid;
wherein the sanitization apparatus (10) comprises:
- a support (12) designed to hold the container (11) in a sanitization area (C);
- at least one ejector assembly (13); each ejector assembly (13) being attachable to the support (12) and fixed to it, in operation, and configured to expel a sanitizing fluid under pressure towards the sanitization area (C) to sanitize a container (11) placed therein.

Step B provides for the sanitization area (C) to be subjected to the sanitizing fluid from multiple directions, possibly varying over time, so that the sanitizing fluid intercepts the entire potentially infected or contaminated surface of the container (11), for sanitization thereof.

## Description

### TECHNICAL FIELD

The present invention is in the technical field of handling potentially infected or contaminated waste, i.e. subject to bacterial danger. This type of waste is generally produced by hospital facilities or collected from vessels, therefore in a port environment. Especially in the case of waste treatment in a port environment, it is expected that this waste is rendered inert, i.e. not bacteriologically harmful, on site.

### STATE OF THE ART

One technique used today for the treatment of this type of port waste is incineration or sterilization. However, such traditional solutions do not guarantee an optimal degree of safety in relation to bacterial risks.

In fact, cases of contamination have been found despite the measures taken to keep the risk of bacteriological contamination low.

For this reason, it is intended to propose a solution to achieve increased health security and minimise the risk of bacteriological contamination that may occur, especially but not only, in port areas in relation to waste handled in any installation where bacteriologically hazardous waste is produced, such as hospitals.

### DEFINITIONS

In this text "infected" means a product or substance containing pathogens that may be harmful to human, animal or plant life.

The term "polluted" or "contaminated" is generally understood to mean an object or fluid that has been exposed to infected material and may therefore be a carrier of it.

### TECHNICAL PROBLEM

The present solution aims to solve the problems highlighted in the prior art by making available an apparatus to increase health and safety and reduce the risk of bacteriological contamination that may occur, especially but not only, in port areas in relation to contaminated waste. The applicant, in fact, noted that upstream of waste incineration or sanitization treatment, there are still large margins of contamination of the environments in which contaminated waste is collected and stored. Not only that, the applicant also noted that, regardless of the effectiveness of the contaminated waste sanitization method, there is a high risk of contamination in the treatment facilities.

The applicant therefore sensed that this contamination might not be directly or solely related to the contaminated waste, but that there might be a real risk from contaminated waste treatment facilities and that, therefore, special care had to be taken in the handling of the contaminated waste and the technical means with which it comes into contact during the treatment chain.

Based on this insight, the applicant found that, surprisingly, limited attention is paid today to contaminated waste transfer devices.

In particular, the present applicant realized that a high degree of risk of contamination could be attributable to lack of (or inadequate) sanitization of the transport containers of contaminated waste packages.

Thanks to this insight, today's applicant realized that within the contaminated waste treatment cycle, a vicious circle takes place whereby there is a cyclical increase in the contamination of waste and the means suitable for transferring it.

### SUMMARY OF THE INVENTION

The invention underpinning this insight involves setting up a sanitization apparatus for a container for transporting potentially infected or contaminated material.

The container, in the traditional way, may have a casing that defines a compartment within it to accommodate loose or packaged waste.
the compartment has an opening that connects the interior and exterior of the casing for filling and emptying the container.

A container can have a lid connected to the casing in an openable and reclosable manner to open and close a compartment opening respectively. Through the opening, the casing can be filled or emptied. The sanitization apparatus may comprise a support designed to hold the container in a sanitization area.

The sanitization apparatus may comprise at least one ejector assembly. Each ejector assembly can be attachable to the support so that it is fixed to the latter when in operation.

Each ejector assembly can be configured to eject a sanitizing fluid towards the sanitization area to sanitize a container placed in it.

The sanitization apparatus according to the present solution can be configured in such a way that, following its activation, the sanitization area is subjected to the sanitizing fluid from multiple directions or from one or more directions varying over time, so that the sanitizing fluid intercepts the entire potentially infected or contaminated surface of the container, for sanitization thereof.

In other words, the sanitization apparatus according to the present solution can be configured in such a way that, following its activation, the sanitization area is subjected to the sanitizing fluid in such a way that it effectively reaches the interior surfaces of the compartment and, where a lid is provided, also the surfaces of the lid.

The sanitization apparatus can be configured in such a way that each ejector assembly is movable around the sanitization area to generate a variable direction for the sanitizing fluid ejected, in operation, into the sanitization area.

For example, the sanitization apparatus may comprise a frame on which one or more ejector assemblies are mounted to surround the support and especially the sanitization area.

The support or frame can be mutually movable so that the container can be sprayed from different directions to optimize the sanitizing fluid reaching all parts of the container.

The sanitization apparatus may comprise a plurality of annular elements surrounding the sanitization area and to which each ejector assembly is fixed.

The annular elements can be movably coupled to the frame, e.g. with rollers, pulleys or guides.

The sanitization apparatus can comprise motor means coupled to the frame and the annular elements to move the ejector assemblies around the sanitization area.

For example, the motor means may comprise, for each annular element, at least one electric motor with a pinion gear that engages, either directly or via a gear motor, with a peripheral gearing provided on each corresponding annular element.

In this way, the drive of the electric motors can cause the annular elements to rotate about the sanitization area during the ejection of the sanitizing fluid, resulting in optimal spraying of the container with the sanitizing fluid.

The ejector assemblies can be rotatable about an operational axis, which can be a central axis common to the annular elements.

According to one or more aspects of the present solution, the sanitization apparatus may comprise a locking assembly operable to firmly secure a container in the sanitization area so that it is not displaced by the sanitizing fluid when subjected to it.

For example, the locking assembly may comprise a framework attached to or forming part of the latter.

The framework could be configured in such a way that the container can be inserted into it and could comprise hooks or restraining elements that can be attached to the container to secure it to the framework. The latter can be reticular or, in any case, formed in such a way as not to impede the flow of sanitizing fluid towards the container.

Structurally, at least one ejector assembly may comprise a tubular manifold element equipped, along its predominant extension direction, with a plurality of through holes designed to connect the interior and exterior of the tubular manifold to eject sanitizing fluid introduced into it. For example, the tubular manifold could consist of a tube with a plurality of side holes that can be engaged by nozzles.

Thus, the injection of sanitizing fluid into the tubular manifold would result in the ejection of the same fluid through the holes or through the nozzles that would be directed towards the sanitization area.

The sanitization apparatus may comprise a hydraulic circuit connected to each ejector assembly and pumping means designed to hydraulically supply pressurized sanitizing fluid to each sanitizing assembly.

According to at least one aspect of the present solution, the sanitization apparatus may comprise heating means for the sanitizing fluid in such a way that the sanitizing fluid to which the container is subjected performs thermal sanitization thereof.

The sanitizing fluid can be an aqueous liquid and the heating means can be configured in such a way that, in operation, the sanitizing fluid is applied to the sanitization area at a temperature substantially comprised between 120°C and 150°C, for a duration which can be comprised between 4 and 10 minutes and can be set to decrease as said set temperature increases.

Said temperature can be comprised between 130°C and 140°C.

Preferably, said temperature is around 134°C and a spraying time of about 6 minutes is envisaged in order to optimize the sanitizing effect with respect to energy and water consumption.

This minimises the consumption of sanitizing fluid, the energy consumption to heat it, and also minimises the amount of sanitizing fluid to be treated following the sanitization of the container.

According to at least one further aspect of the present solution, a method of sanitizing a container for transporting potentially infected or contaminated material may comprise a step A of housing the container in the sanitization area of a sanitization apparatus, followed by a step B of spraying the container with the sanitizing fluid.

As mentioned above, the sanitizing fluid used in the method according to the present solution may be aqueous and, if necessary, may be atomized or ejected in vapour form.

In accordance with the sanitization method according to the present solution, the flow volume of fluidizer sprayed, its temperature and the time for which it is sprayed are selected so as to optimize the sanitizing effect with respect to energy and water consumption or, more generally, consumption of sanitizing fluid.

The sanitizing method according to the present solution may comprise a step C, subsequent to step A, in which each ejector assembly and container may be subjected to a relative movement to generate a variable direction in which the sanitizing fluid ejected, in operation, into the sanitization zone affects a container placed in the latter.

In accordance with the foregoing, the sanitizing method according to the present solution may envisage that in step B the sanitizing fluid affects the sanitization area at a temperature substantially comprised between 120°C and 150°C, which may be comprised between 130°C and 140°C and preferably is around 134°C for spraying the sanitization area comprised between 4 and 10 minutes, as said temperature decreases.

Preferably, in accordance with the present solution, the sanitizing method requires the temperature of the sanitizing fluid to which the container is subjected to be about 134°C for a time of about 6 minutes, in order to optimize the sanitizing effect with respect to energy and water consumption.

It is therefore understood that through the present solution, a sanitization apparatus and/or method for sanitizing a container for transporting potentially infected or contaminated material, the risk of contamination attributable to containers used for collecting, storing and handling potentially infected or contaminated material is greatly reduced. In fact, with the adoption of the present solution, it is understood how recursive contamination of waste is preventable.

In other words, it can be prevented that polluting and/or infected and/or contaminating substances that have fouled a container into which they have been temporarily transferred, contaminate further waste that may subsequently be placed in the same container.

Thanks to the intuition of the present applicant, the present solution makes it possible to significantly reduce the amount of pollutants and/or infectious loads and/or contaminants of the waste transported in the containers, avoiding the contribution of waste contamination by the container itself.

Further features and advantages of the invention will become more apparent from the description of a preferred, but not exclusive, embodiment of a process and apparatus for recycling a solar panel according to the present solution, illustrated for illustrative and nonlimiting purposes in the accompanying drawings, in which:
- Figure 1 illustrates a simplified diagram of a container for transporting potentially infected or contaminated material;
- Figure 2 shows a partial schematic view of a sanitization apparatus for a container for transporting potentially infected or contaminated material;
- Figure 3 shows a schematic perspective view of a sanitization apparatus and a container for transporting potentially infected or contaminated material from Figures 1 and 2;
- Figure 4 shows a schematic view of a sanitization apparatus for a container for transporting potentially infected or contaminated material.

With particular reference to the above-mentioned figures, a purification apparatus for a container 11 for the transport of potentially infected or contaminated material is referred to overall as 10, where the container 11 has a casing 111 that defines an inner compartment A suitable for accommodating loose or packaged waste.

The compartment A has an opening B that connects the interior and exterior of the casing 111 for filling and emptying the container 11.

The container 11 may have one or more lids 111a suitable to close the opening, which are opened when the container 11 is loaded into the sanitization apparatus 10.

The sanitization apparatus 10 comprises a support 12 designed to hold the container 11 in a sanitization area C which, in the example of the accompanying figures, may comprise a shelf or a pair of rails and possibly a ramp 121 providing access for the container 11 to the sanitization area C.

The sanitization apparatus 10 comprises at least one ejector assembly 13 configured to expel a sanitizing fluid towards the sanitization area C. Each ejector assembly 13 is attached to the support 12 preferably in a mutually movable manner, as detailed below.

The sanitization apparatus 10 is configured in such a way that, following its activation, the sanitization area C is subjected to the sanitizing fluid from multiple directions or from one or more directions varying over time, so that the sanitizing fluid intercepts the entire potentially infected or contaminated surface of the container 11, for sanitization thereof.

In particular, each ejector assembly 13 is movable -for example by rotation- around the sanitization area C to generate a direction which varies over time in which the sanitizing fluid is ejected, in operation, towards a container 11 housed in the sanitization area C.

In detail, the sanitization apparatus 10 may comprise
- a frame 101 to which the support 12 is fixed;
- a plurality of annular elements 102 surrounding the sanitization area C and to which each ejector assembly 13 is fixed;
- motor means; coupled to the frame 101 and the annular elements 102 to move the ejector assemblies around the sanitization area C, the annular elements 102 being movably coupled to the frame 101.

In particular, the sanitization apparatus 10 comprises a plurality of ejector assemblies 13 which, as in the example of the accompanying figures, surround the sanitization area C.

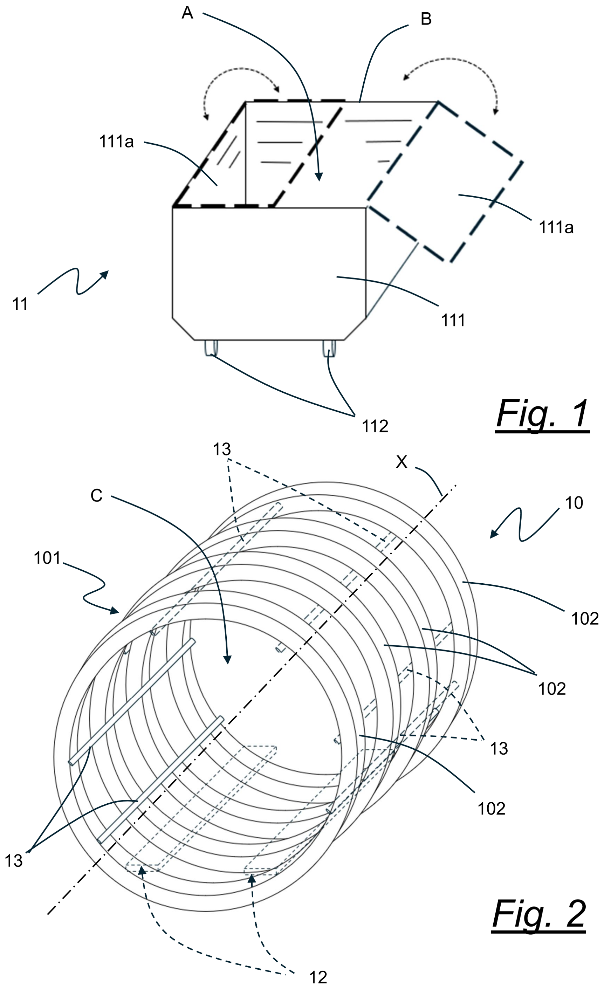

The ejector assemblies 13 are rotatable about an operational axis X, which is a central axis common to the annular elements 102 that is the axis with respect to which they extend circumferentially as in Figures 2-4. A collection basin 20 can be placed below the sanitization area C to collect the sanitizing fluid after it has treated the container 11 so that it can in turn be purified.

The container 11, in the example in the figures, has wheels or skids 112 fixed underneath the casing 111 for moving the container 11.

In order to retain the container 11 following its placement in the sanitization area C, the sanitization apparatus 10 comprises a locking assembly operable to firmly secure a container 11 in the sanitization area C.

The locking assembly may comprise a framework 103 attached to or forming part of the latter.

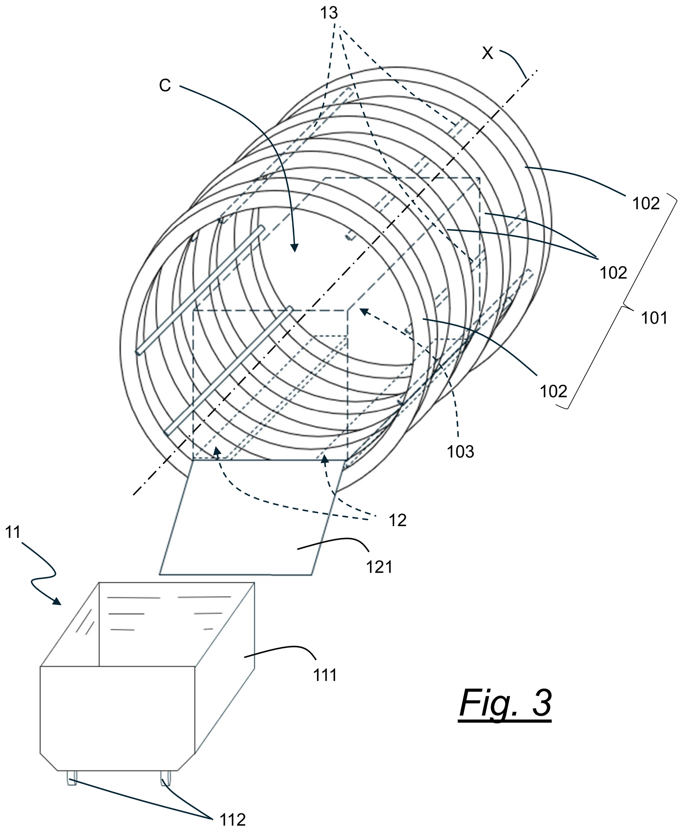

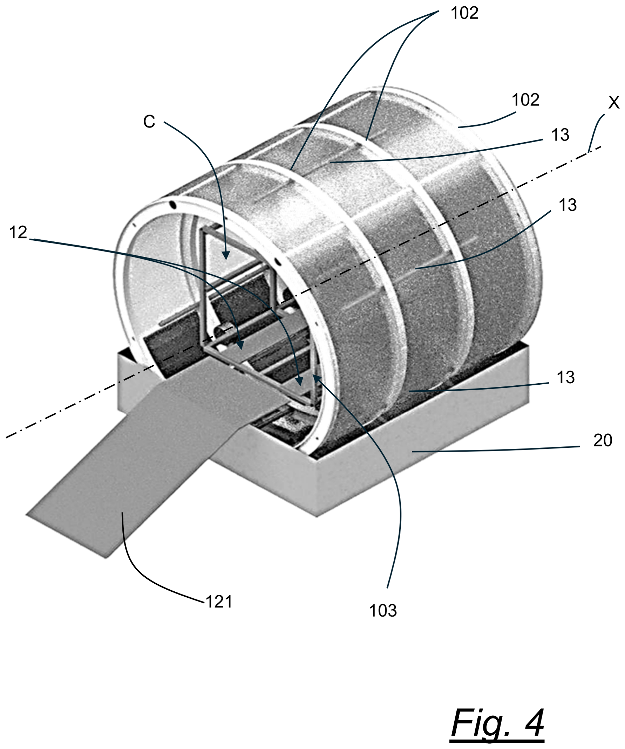

The framework 103, as in the example of Figures 3 and 4, may be configured so that the container may be inserted therein and may comprise hooks or other locking or restraining elements that may be attached to the container to firmly secure it to the framework 103, for example straps.

The framework 103 in Figures 3 and 4 is parallelepiped but, of course, it can be of any shape, e.g. reticular, as long as, in any case, it is formed in such a way that it does not impede the flow of sanitizing fluid towards the container 11 placed in the sanitization area C.

Structurally, the ejector assemblies 13 may comprise, as in the example of Figures 2-4, a tubular manifold element equipped, along its predominant extension direction, with a plurality of through holes designed to connect the interior and exterior of the tubular manifold to eject sanitizing fluid introduced into it.

In an alternative form, not illustrated, the ejector assemblies 13 may comprise a plurality of ejector nozzles, each fed by a sanitizing fluid supply pipe and attached to the frame 101 and oriented towards the sanitization area C.

The sanitization apparatus 10 may comprise a hydraulic circuit connected to each ejector assembly 13 and pumping means, *per se* known and not illustrated in the accompanying figures, capable of hydraulically feeding pressurised sanitizing fluid into each ejector assembly 13.

The sanitization apparatus 10 comprises means of heating the sanitizing fluid, which are in themselves traditional and not illustrated.

The sanitizing fluid is preferably an aqueous liquid, and the heating means are configured in such a way that, in operation, the sanitizing fluid enters the sanitization area C at a temperature substantially comprised between 120°C and 150°C, which can be comprised between 130°C and 140°C, and preferably around 134°C.

A method for sanitizing a container 11 for transporting potentially infected or contaminated material also forms the subject matter of the present solution, which comprises:
- step A, of housing the container 11 in the sanitization area C of the sanitization apparatus 10;
- a step B, subsequent to step A, of spraying the container 11 with the sanitizing fluid, which may preferably be aqueous and possibly atomized or ejected, even in the form of steam.

In accordance with the present solution, the flow volume of fluidizer sprayed, its temperature and the time for which it is sprayed are selected so as to optimize the sanitizing effect with respect to energy and water consumption.

For example, step B requires the sanitizing fluid to enter the sanitization area C at a temperature substantially comprised between 120°C and 150°C, which can be comprised between 130°C and 140°C, and preferably around 134°C.

The spraying time of the sanitization area C is comprised between 3-4 minutes and 10-12 minutes, in general, and is set to decrease with respect to the temperature.

That is, for example, it will be set shorter if the temperature is higher and vice versa.

In particular, the applicant realized that at a temperature of about 134°C and a spraying time of about 6 minutes, it is possible to optimize the sanitizing effect with respect to energy consumption, for heating and pumping, and of sanitizing fluid.

The method for sanitizing a container 11 may comprise a step C, following step A, in which each ejector assembly 13 is rotated with respect to the container 11.

Clearly, in general, they can be subjected to a relative movement to generate a variable direction in which the container 11 is subjected to the ejected sanitizing fluid, optimising the sanitization thereof.

It can therefore be understood how the present solution achieves the predefined tasks and aims and overcomes the problems of the previous state of the art.

The invention thus conceived is susceptible to several modifications and variations, all falling within the scope of protection of the appended claims.

Furthermore, all the details can be replaced by other technically equivalent elements. In practice, the materials used, as well as the contingent shapes and dimensions, may be varied according to requirements and to the state of the art.

Where the construction characteristics and techniques mentioned in the following claims are followed by reference marks or numbers, such reference marks or numbers have been affixed for the sole purpose of increasing the intelligibility of the claims and, consequently, they do not constitute in any way a limitation on the interpretation of each element identified, by way of example only, by such reference marks or numbers.

## Claims

1. Sanitization apparatus (10) for a container (11) for the transport of potentially infected or contaminated material, where the container (11) has a casing (111) defining an inner compartment (A) suitable for accommodating loose or packaged waste; wherein the compartment (A) has an opening (B) that connects the interior and exterior of the casing (111) for filling and emptying the container (11); the sanitization apparatus (10) comprises:
- a support (12) designed to hold the container (11) in a sanitization area (C);
- at least one ejector assembly (13); each ejector assembly (13) being attachable to the support (12) and fixed to it, in operation, and configured to expel a sanitizing fluid towards the sanitization area (C) to sanitize a container (11) placed therein;
where the sanitization apparatus (10) is configured so that, upon activation, the sanitization area (C) is subjected to the sanitizing fluid from multiple directions or from one or more directions varying over time, so that the sanitizing fluid intercepts the entire potentially infected or contaminated surface of the container (11), for sanitization thereof.

2. Sanitization apparatus (10) according to claim 1, configured such that each ejector assembly (13) is movable around the sanitization area (C) to generate a variable direction for the sanitizing fluid ejected, in operation, in the sanitization area (C).

3. Sanitization apparatus (10) according to claim 2, comprising:
a frame (101) to which the support (12) is fixed;
a plurality of annular elements (102) surrounding the sanitization area (C) and to which each ejector assembly (13) is fixed; the annular elements (102) being movably coupled to the frame (101);
motor means coupled to the frame (101) and the annular elements (102) to move the ejector assemblies (13) around the sanitization area (C).

4. Sanitization apparatus (10) according to claim 3, wherein each ejector assembly (13) is rotatable around an operational axis (X), which is a central axis common to the annular elements (102).

5. Sanitization apparatus (10) according to any of the preceding claims, comprising a locking assembly operable to firmly secure a container (11) in the sanitization area (C) so that it is not displaced by the sanitizing fluid when subjected to it.

6. Sanitization apparatus (10) according to any of the preceding claims, where at least one ejector assembly (13) comprises a tubular manifold element equipped, along its predominant extension direction, with a plurality of through holes designed to connect the interior and exterior of the tubular manifold to eject sanitizing fluid introduced into it; where the sanitization apparatus (10) comprises a hydraulic circuit connected to each ejector assembly (13) and pumping means designed to hydraulically supply pressurized sanitizing fluid to each ejector assembly (13).

7. Sanitization apparatus (10) according to any of the preceding claims, comprising heating means for the sanitizing fluid, which is preferably an aqueous liquid, where the heating means are configured so that, in operation, the sanitizing fluid is applied to the sanitization area (C) at a temperature substantially between 120°C and 150°C, optionally between 130°C and 140°C, and preferably at approximately 134°C for an application duration of about 6 minutes to optimize the sanitizing effect relative to energy and water consumption.

8. Method for sanitizing a container (11) designed for the transport of potentially infected or contaminated material, comprising:
- step A: positioning the container (11) in the sanitization area (C) of a sanitization apparatus (10) according to any of the preceding claims;
- step B: subsequent to step A, spraying the container (11) with the sanitizing fluid, which may be aqueous and optionally nebulized or ejected in the form of steam; wherein the flow volume, temperature, and duration of the fluid application are selected to optimize the sanitizing effect relative to energy and water consumption.

9. Method for sanitizing a container (11), according to claim 8, comprising a step C, subsequent to step A, wherein each ejector assembly (13) and the container (11) are subject to relative movement to generate a variable direction for the sanitizing fluid ejected, in operation, in the sanitization area (C) and applied to the container (11) placed therein.

10. Method for sanitizing a container (11), according to claims 8 or 9,
where step B provides for the sanitizing fluid to be applied to the sanitization area (C) at a temperature substantially between 120°C and 150°C, optionally between 130°C and 140°C, and preferably at approximately 134°C for an application duration of 4 to 10 minutes, decreasing with temperature; wherein, if the temperature is approximately 134°C, the application time lasts about 6 minutes to optimize the sanitizing effect relative to energy and water consumption.
